(19) Europäisches Patentamt / European Patent Office / Office européen des brevets

(11) **EP 3 958 737 B1**

(12) **EUROPEAN PATENT SPECIFICATION**

(45) Date of publication and mention of the grant of the patent:
**12.03.2025 Bulletin 2025/11**

(21) Application number: **20723795.9**

(22) Date of filing: **20.04.2020**

(51) International Patent Classification (IPC):
**A61B 5/11** *(2006.01)*    **A61B 5/113** *(2006.01)*
**A61B 5/00** *(2006.01)*

(52) Cooperative Patent Classification (CPC):
**A61B 5/113; A61B 5/1116; A61B 5/1123; A61B 5/72;** A61B 2562/0219

(86) International application number:
**PCT/EP2020/060944**

(87) International publication number:
**WO 2020/216694 (29.10.2020 Gazette 2020/44)**

(54) **A DEVICE, SYSTEM, AND METHOD FOR MEASURING PHYSIOLOGICAL PHENOMENA AND PHYSICAL ACTIVIVIES IN A SUBJECT**

VORRICHTUNG, SYSTEM UND VERFAHREN ZUR MESSUNG PHYSIOLOGISCHER PHÄNOMENE UND PHYSIKALISCHER AKTIVITÄTEN IN EINEM SUBJEKT

DISPOSITIF, SYSTÈME ET PROCÉDÉ DE MESURE DE PHÉNOMÈNES PHYSIOLOGIQUES ET D'ACTIVIATIONS PHYSIQUES CHEZ UN SUJET

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **23.04.2019 US 201962837448 P**

(43) Date of publication of application:
**02.03.2022 Bulletin 2022/09**

(73) Proprietor: **Koninklijke Philips N.V.**
**5656 AG Eindhoven (NL)**

(72) Inventors:
• **THIAGARAJAN, Srik**
 **5656 AE Eindhoven (NL)**
• **GAO, Mingwu**
 **5656 AE Eindhoven (NL)**

(74) Representative: **Philips Intellectual Property & Standards**
**High Tech Campus 52**
**5656 AG Eindhoven (NL)**

(56) References cited:
WO-A1-2016/096454    WO-A2-03/005879
US-A1- 2018 146 922    US-A1- 2019 053 754

• VINAYAGA-SURESHKANTH NISHA ET AL: "Towards a Practical Pedestrian Distraction Detection Framework using Wearables", 2018 IEEE INTERNATIONAL CONFERENCE ON PERVASIVE COMPUTING AND COMMUNICATIONS WORKSHOPS (PERCOM WORKSHOPS), IEEE, 19 March 2018 (2018-03-19), pages 239 - 245, XP033414112, DOI: 10.1109/PERCOMW.2018.8480238
• W.A SETHARES ET AL: "Periodicity transforms", IEEE TRANSACTIONS ON SIGNAL PROCESSING, 1 November 1999 (1999-11-01), pages 2953 - 2964, XP055717323, Retrieved from the Internet <URL:https://sethares.engr.wisc.edu/paperspdf/pertrans.pdf> DOI: 10.1109/78.796431
• ANNAPURNA SHARMA ET AL: "Frequency based classification of activities using accelerometer data", MULTISENSOR FUSION AND INTEGRATION FOR INTELLIGENT SYSTEMS, 2008. MFI 2008. IEEE INTERNATIONAL CONFERENCE ON, IEEE, PISCATAWAY, NJ, USA, 20 August 2008 (2008-08-20), pages 150 - 153, XP031346262, ISBN: 978-1-4244-2143-5

**Description**

**Field of the Disclosure**

The present disclosure relates to a device, system, and method for measuring physiological phenomena and physical activities associated with a subject. Specifically, the present disclosure relates to determining a dominant activity observed from body-worn sensors and accurately characterizing that activity.

**Background of the Disclosure**

The health care industry is experiencing an emergence of wireless technologies that may replace conventional health monitoring devices with wearable devices. These wearable devices may monitor physiological data related to the health of a patient. As a result, these wearable devices may vastly improve disease prevention, clinical care, and quality of life.

However, obtaining clinically-accurate data from these wearable devices is proving quite difficult. For example, wearable medical devices may attempt to isolate useful physiological information from data gathered via a device worn on the body. In order to accomplish this goal, devices may employ a number of components including, for example, an accelerometer, gyrometer or gyroscope, magnetometer, and/or barometer. The physiological information may include, for example, pulse rate, blood pressure, respiratory rate, and/or temperature. Among this physiological information, respiratory rate is often not recorded or is inaccurate and therefore, unusable for adverse event detection.

For example, respiratory rate measurements may contain inaccuracies when based on periodic and aperiodic chest wall movements and vibrations. Mechanical motions as seen from periodic and aperiodic chest wall movements and vibrations may be defined as detection of dominant visible activity at any particular time. And, for a patient at rest, sensors may indicate a baseline respiration activity along with sensor noise that is aperiodic. When a patient rises to a standing or sitting position or otherwise changes posture, sudden aperiodic changes may manifest in sensor data. And, when a patient is mobile, such as when a patient is walking, a set of periodic oscillations may be seen. At any point in time, the ratio of the dominant activity versus the other activities may determine a signal quality index (SQI) measurement. And, in order to make use of measurements such as respiratory rate or other activity types, an assurance of quality via a quality index may be required to accept measurements that can be reliably used. Present models for respiratory rate do not provide high-quality SQI measurements, and thereby do not provide clinically accurate respiratory rate measurements. For example, when the dominant activity is not properly determined an accurate respiratory rate cannot be calculated.

But, activity levels are not often accurately determined. For example, medical quality algorithms may use measurements and/or context information that require an assurance or a quality index to obtain acceptable measurements that can be reliably used. Moreover, current models do not employ an accurate or near accurate signal quality index to use along with each measurement.

Further complications of existing methods include problems associated with overlapping activities related to, for example, respiration, slow walking cycles, posture changes, and/or other motion-related artifacts. These complications are evidenced by an inability to provide one-to-one relations that associate frequency domain information with a particular physiological phenomenon.

In addition to these problem is the fact that respiratory rate is often not recorded, even when the patient's primary problem is a respiratory condition. This is also in spite of the fact that an abnormal respiratory rate may be an important predictor of serious events such as cardiac arrest or admission to an intensive care unit (ICU). In fact, higher respiration rate in patients may been associated with increased mortality. These and other drawbacks exist.

Advantageously, the present invention seeks to solve these outstanding problems by measuring physiological phenomena and physical activities happening at any point in a subject's body.

Vinayaga-Sureshkanth, Nisha et al,"Towards a Practical Pedestrian Distraction Detection Framework using Wearables", 2018 IEEE International Conference on Pervasive Computing and Communications Workshops (PerCom Workshops), IEEE, 19 March 2018, pages 239-245 discloses a wearable device that determines whether a pedestrian user is distracted through signal processing of accelerometer and/or gyroscope data using frequency domain information such as the fast Fourier transform.

Sethares, W.A. and Staley. T.W.: "Periodicity transforms", IEEE Transactions on Signal Processing, 1 November 1999, pages 2953-2964 discloses a method of detecting periodicities in data that exploits a series of projections onto "periodic subspaces". The periodicity transform does not assume a fixed predetermined basis as in the Fourier transform, and is linear-in-period rather than linear-in-frequency or linear-in-scale. Examples including the separation of periodic waveforms with overlapping spectra are given and compared to the Fourier transform.

## Summary of the Disclosure

The invention is defined by the claims. According to various embodiments, the present disclosure relates to a method to determine the dominant activity observed from body-worn sensors and accurately characterize the activity. Various embodiments may include using Periodicity Transform (PT) along with Partial Cycle Measurement (PCM) calculations to detect a dominant activity associated with a data interval from one or more data channels.

For example, in a body-worn sensor (e.g., chest-worn, limb-worn, etc.), there may be contradictory signals that overlap in time, frequency, amplitudes, and/or the like, and this overlapping data may convey the specific dominant information in a mixed way. Various embodiments described herein seek to determine the dominant source of information from any channel by using fractional cycles and periodicity transforms. For example periodicity transforms according to various embodiments may provide specific transformation methods that are implementable in real-time and identify the dominant cycle period when used along with heuristics to eliminate harmonic/sub-period information. Accordingly, the various embodiments described herein may determine a dominant activity happening inside or with the patient along with a reliability measure (e.g., signal-quality index) for the particular measurement interval.

By way of example, respiratory rate measurements such as a measurement of various mechanical motions as seen from periodic and aperiodic chest wall movements and vibrations, which can be defined as detection of dominant visible activity at any particular time. For a patient at rest, sensors may indicate a baseline respiration activity along with sensor noise, which is aperiodic. When a patient gets up, or changes posture, or turns around, sudden aperiodic changes manifest in sensor data. Again, a set of periodic oscillations may be seen, when a patient goes through a regular slow or quick walk. At any instant in time, the ratio of the dominant activity versus the rest of the activity can determine the signal quality index (SQI) measure.

Various embodiments of the disclosure may include a device having an accelerometer configured to receive accelerometer data; a gyroscope configured to receive gyroscope data; and a microprocessor configured to: perform partial cycle analysis to determine at least a top three cycles based on at least one of: the accelerometer data and gyroscope data; perform a periodicity transform (PT)-based selection of at least one top periodicity observed from the partial cycle analysis; and determine a most likely activity of the at least one top periodicity using heuristics. In various embodiments, the most likely activity may include at least one of: sudden motion, sustained motion, slow chest wall movement, deep chest wall movement, and vigorous vibration.

According to various embodiments, PT-based selection may be performed by: (a) assuming a threshold (T); (b) obtaining signal X of length N via at least one of: the accelerometer data and the gyroscope data; (c) locating a fractional cycle marker for each cycle of the at least top three cycles; (d) approximating a cycle length ($CL_i$) and an amplitude ($A_i$) for each cycle, wherein i is a cycle; (e) approximating the expected periodicities for each cycle; (f) relating the expected periodicities to the fractional cycle marker for each cycle; (g) letting an expected periodicity equal to one; (h) removing a linear DC component by removing the projection onto the first expected periodicity equal to one; (i) letting the expected periodicity equal $CL_1$; (j) determining whether $A_1$ contains at least T percentage of energy in X; (k) if $A_1$ contains at least T percentage of energy in X, accepting $A_1$ and $CL_1$ as a possible cycle; (l) repeating steps (i)-(k) for each $CL_i$ and $A_i$; and (m) estimating a most likely cycle length based on each cycle length ($CL_i$) and an amplitude ($A_i$) for each cycle.

According to various embodiments, partial cycle estimation may occur simultaneous to or separately from periodicity transformation estimations.

In various embodiments, if adjacent periodicities show dominant characteristics due to a variability inside the measurement interval, periodicity bands are formed, and a center periodicity inside a band is nominated as the most likely cycle length. And, in various embodiments heuristics may be based on at least one of: a particular amplitude range, a particular cycle length, data indicative of continuity, data indicative of a duration of continuity, data indicative of a periodic nature, data indicative of what channels of sensors a signal is dominant in, and a degree of correlation with measurements made in silent intervals. According to various embodiments heuristics may include at least one of: a maximum power cycle length associated with at the accelerometer data, a maximum power cycle length associated with the gyroscope data, a mean power cycle length associated with at the accelerometer data, a mean power cycle length associated with the gyroscope data, a maximum significant estimated cycle length associated with at the accelerometer data, a maximum significant estimated cycle length associated with the gyroscope data.

Also, according to various embodiments, a microprocessor may measure a signal quality index (SQI) for an incoming signal associated with at least one of the accelerometer data and gyroscope data using the presence or absence of periodicity information associated with the partial cycle analysis. Accordingly to various embodiments a sudden change in SQI for a duration less than a first SQI threshold may indicate a posture change.

In various embodiments, the accelerometer and gyroscope may be housed in a wearable device, which may be worn on a user's chest, either in the upper chest or lower chest regions. A user may include a patient. In various embodiments, accelerometer data and/or gyroscope data may be received on-demand and/or continuously. In various embodiments, sensor data may be pushed from a wearable device and/or pulled from a wearable device.

According to various embodiments a microprocessor may determine a signal quality index (SQI) associated with the most likely activity.

In various embodiments, a partial cycle analysis may be performed by mapping cycles onto regions of maximum amplitudes, minimum amplitudes, and zero crossing intervals and their periodicities. This mapping may be continuous. And, in various embodiments, a cycle may be selected as an at least top three cycles based on amplitude thresholds and repeatability of periodicity. Moreover, in various embodiments a baseline measurement noise amplitude may be computed and subtracted from amplitude measurements associated with at least one of accelerometer data and gyroscope data.

Various embodiments my include receiving accelerometer data via an accelerometer of a wearable device; receiving gyroscope data via a gyroscope of the wearable device; performing partial cycle analysis to determine at least a top three cycles based on at least one of: the accelerometer data and gyroscope data; performing a periodicity transform (PT)-based selection of at least one top periodicity observed from the partial cycle analysis; and determining a most likely activity of the at least one top periodicity using heuristics. Various embodiments may include a most likely activity of respiration and a top periodicity of a respiration rate. Moreover, various embodiments may conclude that a most likely activity includes at least one of: sudden motion, sustained motion, slow chest wall movement, deep chest wall movement, and vigorous vibration.

Various embodiments may include performing a PT-based selection is by: (a) assuming a threshold (T); (b) obtaining signal X of length N via at least one of: the accelerometer data and the gyroscope data; (c) locating a fractional cycle marker for each cycle of the at least top three cycles; (d) approximating a cycle length ($CL_i$) and an amplitude ($A_i$) for each cycle, wherein i is a cycle; (e) approximating the expected periodicities for each cycle; (f) relating the expected periodicities to the fractional cycle marker for each cycle; (g) letting an expected periodicity equal to one; (h) removing the linear DC component by removing the projection onto the first expected periodicity equal to one; (i) letting the expected periodicity equal $CL_1$; (j) determining whether $A_1$ contains at least T percentage of energy in X; (k) if $A_1$ contains at least T percentage of energy in X, accepting $A_1$ and $CL_1$ as a possible cycle; (l) repeating steps (i)-(k) for each $CL_i$ and $A_i$; and (m) estimating a most likely cycle length based on each cycle length ($CL_i$) and an amplitude ($A_i$) for each cycle.

In various embodiments the implementation of any methods or device-based algorithms may be in the form of hardware and/or firmware. For example a low pass filter associated with a particular signal may filter or pass one or more signals with a frequency lower than a predefined threshold frequency. Advantageously, by placing filters in hardware, the filters may be sturdy.

Various embodiments of the present disclosure provide a device, system, and method for measuring physiological phenomena and physical activities associated with a subject. In various embodiments,

## Brief Description of the Drawings

Various embodiments of the present disclosure, together with further objects and advantages, may best be understood by reference to the following description taken in conjunction with the accompanying drawings, in the several Figures of which like reference numerals identify like elements, and in which:

Figure 1 depicts an example embodiment of a system including device that measures physiological phenomena and physical activities associated with a subject;

Figures 2 depicts an example embodiment of a system including device that measures physiological phenomena and physical activities associated with a subject;

Figure 3 depicts an example embodiment of a system including device that measures physiological phenomena and physical activities associated with a subject;

Figure 4 depicts a flow diagram that illustrates an example method that measures physiological phenomena and physical activities associated with a subject in accordance with an embodiment of the invention;

Figure 5 depicts an example device housing technology that measures physiological phenomena and physical activities associated with a subject in accordance with an embodiment of the invention; and

Figure 6 depicts an example device housing technology that measures physiological phenomena and physical activities associated with a subject in accordance with an embodiment of the invention.

## DETAILED DESCRIPTION OF THE EMBODIMENTS

The following description is intended to convey a thorough understanding of the embodiments described by providing a number of specific exemplary embodiments and details involving a device, system, and method for measuring physiological phenomena and physical activities associated with a subject, such as measuring signal data, determining a signal quality index, and/or determining a most likely activity associated with sensor data. It should be appreciated, however, that the present disclosure is not limited to these specific embodiments and details, which are examples only. It is further understood that one possessing ordinary skill in the art, in light of known systems and methods, would appreciate the use of the invention for its intended purposes and benefits in any number of alternative embodiments, depending on specific design and other needs.

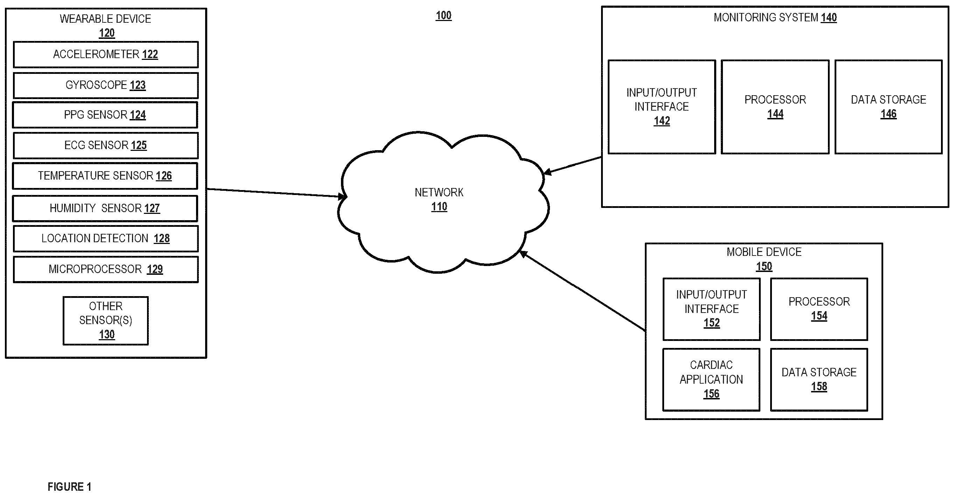

Figure 1 depicts an exemplary system 100 for use with the device, system, and method for measuring physiological phenomena and physical activities associated with a subject, such as measuring signal data, determining a signal quality index, and/or determining a most likely activity associated with sensor data. As shown in Figure 1, an example system 100 may include one or more wearable devices 120, one or more monitoring systems 140, and/or one or more mobile devices 150 connected over one or more networks 110.

For example, network 110 may be one or more of a wireless network, a wired network or any combination of wireless network and wired network. For example, network 110 may include one or more of a fiber optics network, a passive optical network, a cable network, an Internet network, a satellite network, a wireless LAN, a Global System for Mobile Communication ("GSM"), a Personal Communication Service ("PCS"), a Personal Area Network ("PAN"), Wireless Application Protocol (WAP), Multimedia Messaging Service (MMS), Enhanced Messaging Service (EMS), Short Message Service (SMS), Time Division Multiplexing (TDM) based systems, Code Division Multiple Access (CDMA) based systems, D-AMPS, Wi-Fi, Fixed Wireless Data, IEEE 802.11b, 802.15.1, 802.11n and 802.11g, a Bluetooth network, or any other wired or wireless network for transmitting and receiving a data signal.

In addition, network 110 may include, without limitation, telephone lines, fiber optics, IEEE Ethernet 902.3, a wide area network ("WAN"), a local area network ("LAN"), a wireless personal area network ("WPAN"), a wireless body area network ("WBAN"), or a global network such as the Internet. Also network 110 may support an Internet network, a wireless communication network, a cellular network, or the like, or any combination thereof. Network 110 may further include one network, or any number of the example types of networks mentioned above, operating as a stand-alone network or in cooperation with each other. Network 110 may utilize one or more protocols of one or more network elements to which they are communicatively coupled. Network 110 may translate to or from other protocols to one or more protocols of network devices. Although network 110 is depicted as a single network, it should be appreciated that according to one or more embodiments, network 110 may comprise a plurality of interconnected networks, such as, for example, the Internet, a service provider's network, a cable television network, corporate networks, and home networks.

Wearable device 120 may include, for example, one or more accelerometer 122, one or more gyroscope 123, on or more photoplethysmography (PPG) sensor 124, one or more electrocardiogram (ECG) sensors 125, one or more temperature sensor 126, one or more humidity sensor 127, location-detection technology 128, and/or one or more microprocessor 129. Wearable device may also include other sensors 130 such as any magnetometer or barometer.

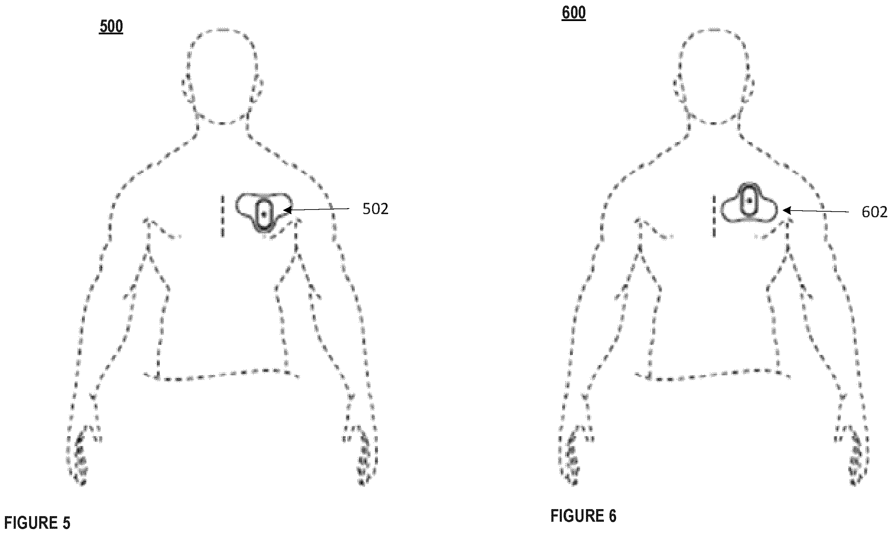

A wearable device 120 may, for example, receive signals and/or data indicative of: a vital sign (e.g., heart rate, respiratory rate, temperature, blood pressure, blood glucose level, pulse oximetry, and/or the like), a pain measurement, a stress measurement (e.g., via vital signs, skin conductance data, and/or the like), an activity measurement (e.g., a number of steps taken, a number of floors climbed, a type of activity, a distance a user has moved, a gait characteristic, a gait speed, and/or the like). A wearable device 120 may be a limb-worn device (e.g., wrist, arm, ankle, leg, etc.), a chest-worn device, and/or a multi-location worn device. In various embodiments, an ECG device 110 and/or wearable device 120 may comprise a wireless patch, a wired patch, a limb-worn device, and/or an implantable device. By way of example, a wearable device may be a chest worn patch as illustrated in Figures 5 and 6. For example, a chest worn patch 502, 602 may be worn on a user/patient 500/600 as illustrated. A chest worn patch 502, 602 may have any shape and/or may be placed on the chest in any manner.

Monitoring system 140 may include an input/output interface 142, a processor 144, and/or data storage 146. Although monitoring system 140 is depicted as a standalone system, monitoring system 140 may be incorporated into, for example a wearable device 120 and/or a mobile device 150.

Monitoring system 140 and/or mobile device 150 may include, for example, one or more mobile devices, such as, for example, personal digital assistants (PDA), tablet computers and/or electronic readers (e.g., iPad®, Kindle Fire®, Playbook, Touchpad, etc.), wearable devices (e.g., Google® Glass, Apple Watch®, etc.), telephony devices, smartphones, cameras, music playing devices (e.g., iPod®, etc.), televisions, patient monitoring devices (e.g., Philips Intellivue®, Philips Avalon®, Philips IntelliSpace®, etc.), set-top-box devices, and the like.

Monitoring system 140 and/or mobile device 150 also may include a network-enabled computer system and/or device. As referred to herein, a network-enabled computer system and/or device may include, but is not limited to: *e.g.,* any computer device, or communications device including, *e.g.,* a server, a network appliance, a personal computer (PC), a workstation, a mobile device, a phone, a handheld PC, a personal digital assistant (PDA), a thin client, a fat client, an Internet browser, or other device. The network-enabled computer systems may execute one or more software applications to, for example, receive data as input from an entity accessing the network-enabled computer system, process received data, transmit data over a network, and receive data over a network.

Monitoring system 140 and/or mobile device 150 may include at least one central processing unit (CPU), which may be configured to execute computer program instructions to perform various processes and/or methods. Processes and/or methods as described herein may be understood to refer to computer executable software, firmware, hardware, and/or various combinations thereof. It is noted there where a process and/or method includes a software and/or firmware component, the software and/or firmware is configured to affect the hardware elements of an associated system. It is further noted that the processes and/or methods shown and described herein are intended as examples. The processes and/or methods may be combined, integrated, separated, or duplicated to support various applications. Also, a function described herein as being performed at a particular process and/or method may be performed during one or more other processes and/or methods and by one or more other devices instead of or in addition to the function performed as described. Further, the processes and/or methods may be implemented across multiple devices or other components local or remote to one another. Additionally, the processes and/or methods may be moved from one device and added to another device, or may be included in both devices.

Monitoring system 140 and/or mobile device 150 may include an input/output interface 142, 152. Input/output interface 142, 152 also may include antennas, network interfaces that may provide or enable wireless and/or wire line digital and/or analog interface to one or more networks, such as network 110, over one or more network connections, a power source that provides an appropriate alternating current (AC) or direct current (DC) to power one or more components of system 100, and a bus that allows communication among the various components of system 100. Input/output interface 142, 152 may include a display, which may include for example output devices, such as a printer, display screen (e.g., monitor, television, and the like), speakers, projector, and the like. Although not shown, input/output interface 142, 152 may include one or more encoders and/or decoders, one or more interleavers, one or more circular buffers, one or more multiplexers and/or de-multiplexers, one or more permuters and/or depermuters, one or more encryption and/or decryption units, one or more modulation and/or demodulation units, one or more arithmetic logic units and/or their constituent parts, and/or the like.

Input/output interface 142, 152 may include a Bluetooth module or chipset with a Bluetooth transceiver, a chip, and an antenna. The transceiver may transmit and receive information via the antenna and an interface. The chip may include a microprocessor that stores and processes information specific to a piconet and provides device control functionality. Device control functionality may include connection creation, frequency-hopping sequence selection and timing, power control, security control, polling, packet processing, and the like. The device control functionality and other Bluetooth-related functionality may be supported using a Bluetooth API provided by the platform associated with the monitoring system 140 and/or mobile device 150. Using a Bluetooth API, an application stored on monitoring system 140 and/or mobile device 150 may be able to scan for other Bluetooth devices, query the local Bluetooth adapter for paired Bluetooth devices, establish RFCOMM channels, connect to other devices through service discovery, transfer data to and from other devices, and manage multiple connections. A Bluetooth API used in the methods, systems, and devices described herein may include an API for Bluetooth Low Energy (BLE) to provide significantly lower power consumption and allow monitoring system 140 and/or mobile device 150 to communicate with BLE devices that have low power requirements (e.g., wearable device 120).

Input/output interface 142, 152 may also include an NFC antenna and secure element (SE). In one embodiment, the SE may be used for digitally and physically secure storage of sensitive data. The SE may include a computer processor or other computational hardware or software.

Input/output interface 142, 152 may enable Industry Standard NFC Transmission. For example, the input/output interface 142, 152 may enable two loop antennas to form an air-core transformer when placed near one another by using magnetic induction. Input/output interface 142, 152 may operate at 13.56 MHz or any other acceptable frequency. Also, input/output interface 142, 152 may provide for a passive communication mode, where the initiator device provides a carrier field, permitting answers by the target device via modulation of existing fields. Additionally, input/output interface 142, 152 also may provide for an active communication mode by allowing alternate field generation by the initiator and target devices.

Input/output interface 142, 152 may deactivate an electromagnetic field for wireless broadcasting and/or communication during particular (e.g., predefined and/or otherwise specified) intervals. For example, input/output interface 142, 152 may deactivate an RF field while awaiting data. This deactivation may use Miller-type coding with varying modulations, including 100% modulation. The deactivation may also use Manchester coding with varying modulations, including a modulation ratio of 10%. Additionally, input/output interface 142, 152 may be capable of receiving and transmitting data at the same time, as well as checking for potential collisions when the transmitted signal and received signal frequencies differ.

Input/output interface 142, 152 may be capable of utilizing standardized transmission protocols. Also, input/output module 142, 152 may be able to utilize transmission protocols and methods that are developed in the future using other frequencies or modes of transmission. Input/output module 142, 152 may also be backwards-compatible with existing techniques, for example RFID.

Monitoring system 140 and/or mobile device 150 may include data storage 146, 156, including for example, random access memory (RAM) and read only memory (ROM), which may be configured to access and store data and information and computer program instructions. Data storage 146, 156 may also include storage media or other suitable type of memory (e.g., such as, for example, RAM, ROM, programmable read-only memory (PROM), erasable programmable read-only memory (EPROM), electrically erasable programmable read-only memory (EEPROM), magnetic disks, optical disks, floppy disks, hard disks, removable cartridges, flash drives, any type of tangible and non-transitory storage medium), where the files that comprise an operating system, application programs including, for example, web browser application, email application and/or other applications, and data files may be stored. Data storage 146, 156 may include electronic information, files, and documents stored in various ways, including, for example, a flat file, indexed file, hierarchical database, relational database, such as a database created and maintained with software from, for example, Oracle® Corporation, Microsoft® Excel file, Microsoft® Access file, or any other storage mechanism.

Monitoring system 140 and/or mobile device 150 may further include, for example, a processor 144, 154, which may be several processors, a single processor, or a single device having multiple processors. Processor 144, 154 may include any range of processing, such as, for example a one-chip microprocessor or a multi-chip processing unit. In various embodiments, processor 144 and/or processor 154 may execute a number of instructions and/or modules.

Monitoring system 140 and/or mobile device 150 may include any mobile device running the Apple® iOS operating system (e.g., iPhone, iPad, Apple Watch, and/or the like) and/or any device running Google's Android® operating system, including, for example, smartphones running the Android® operating system and other wearable mobile devices, such as Google® Glass or Samsung Galaxy Gear® Smartwatch , any device running Microsoft's Windows® Mobile operating system, and/or any other smartphone or like device.

Although not shown, mobile device 150, monitoring system 140, ECG device 110 and/or wearable device 120 may connect to a system associated with a general practitioner and/or specialist over network 110 in order to transmit any received data and/or measurements associated with the monitoring described herein.

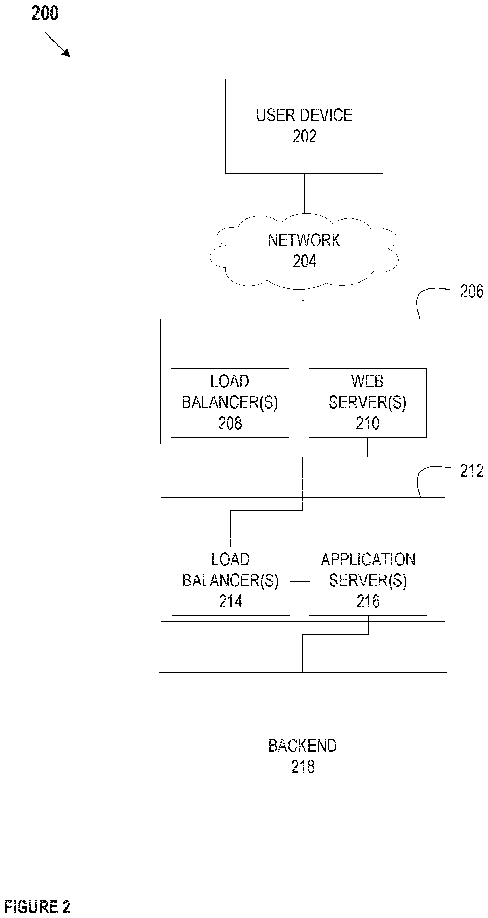

Figure 2 depicts an example system that may be used for patient monitoring as described herein. The example system 200 in Figure 2 may enable a medical facility, for example, to provide networked services and solution to patients, such as vital signs monitoring and analysis as described herein. As shown in Figure 2, system 200 may include a user device 202 (e.g., a wearable device, an implantable device, and/or the like as described herein), a network 204, a front-end controlled domain 206, a back-end controlled domain 212, and a backend 218. Front-end controlled domain 206 may include one or more load balancers 208 and one or more web servers 210. Back-end controlled domain 212 may include one or more load balancers 214 and one or more application servers 216.

User device 202 may include some form of a network-enabled computing device. As referred to herein, a network-enabled computing device may include, but is not limited to: e.g., any computer device, or communications device including, e.g., a wearable device, an implantable device, a thin client, a fat client, an Internet browser, or other device. The one or more network-enabled computing devices of the example system 200 may execute one or more software applications to enable, for example, network communications.

A computing device may include any computer device, or communications device including, e.g., a server, a network appliance, a personal computer (PC), a workstation, a mobile device, a phone, a handheld PC, a personal digital assistant (PDA), a thin client, a fat client, an Internet browser, or other device. These devices may include a monitoring device that receives data from a wearable and/or implantable device. The one or more network-enabled computers of the example system 200 may execute one or more software applications to enable, for example, network communications.

User device 202 also may be a mobile device. For example, a mobile device may include an iPhone, iPod, iPad from Apple® or any other mobile device running Apple's iOS operating system, any device running Google's Android® operating system, including for example, Google's wearable device, Google Glass, any device running Microsoft's Windows® Mobile operating system, and/or any other smartphone or like wearable mobile device.

Network 204 may be one or more of a wireless network, a wired network, or any combination of a wireless network and a wired network. For example, network 204 may include one or more of a fiber optics network, a passive optical network, a cable network, an Internet network, a satellite network, a wireless LAN, a body area network (BAN), a wide area network (WAN), a Global System for Mobile Communication (GSM), a Personal Communication Service (PCS), a Personal Area Networks, (PAN), D-AMPS, Wi-Fi, Fixed Wireless Data, IEEE 802.11b, 802.15.1, 802.11n, and 802.11g or any other wired or wireless network for transmitting and receiving a data signal.

In addition, network 204 may include, without limitation, telephone lines, fiber optics, IEEE Ethernet 902.3, a wide area network (WAN), a local area network (LAN), a body area network (BAN) or a global network such as the Internet. Also, network 204 may support an Internet network, a wireless communication network, a cellular network, or the like, or any combination thereof. Network 204 may further include one network, or any number of example types of networks mentioned above, operating as a stand-alone network or in cooperation with each other. Network 204 may utilize one or more protocols of one or more network elements to which they are communicatively couples. Network 204 may translate to or from other protocols to one or more protocols of network devices. Although network 204 is depicted as a single network, it should be appreciated that according to one or more embodiments, network 204 may comprise a plurality of interconnected networks, such as, for example, the Internet, a service provider's network, a cable television network, corporate networks, and home networks.

Front-end controlled domain 206 may be implemented to provide security for backend 218. Load balancer(s) 208 may distribute workloads across multiple computing resources, such as, for example computers, a computer cluster, network links, central processing units or disk drives. In various embodiments, load balancer(s) 210 may distribute workloads across, for example, web server(S) 216 and/or backend 218 systems. Load balancing aims to optimize resource use, maximize throughput, minimize response time, and avoid overload of any one of the resources. Using multiple components with load balancing instead of a single component may increase reliability through redundancy. Load balancing is usually provided by dedicated software or hardware, such as a multilayer switch or a Domain Name System (DNS) server process.

Load balancer(s) 208 may include software that monitoring the port where external clients, such as, for example, user device 202, connect to access various services and/or devices of a medical facility, for example. Load balancer(s) 208 may forward requests to one of the application servers 216 and/or backend 218 servers, which may then reply to load balancer 208. This may allow load balancer(s) 208 to reply to user device 202 without user device 202 ever knowing about the internal separation of functions. It also may prevent user devices from contacting backend servers directly, which may have security benefits by hiding the structure of the internal network and preventing attacks on backend 218 or unrelated services running on other ports, for example.

A variety of scheduling algorithms may be used by load balancer(s) 208 to determine which backend server to send a request to. Simple algorithms may include, for example, random choice or round robin. Load balancers 208 also may account for additional factors, such as a server's reported load, recent response times, up/down status (determined by a monitoring poll of some kind), number of active connections, geographic location, capabilities, or how much traffic it has recently been assigned.

Load balancers 208 may be implemented in hardware and/or software. Load balancer(s) 208 may implement numerous features, including, without limitation: asymmetric loading; Priority activation: SSL Offload and Acceleration; Distributed Denial of Service (DDoS) attack protection; HTTP/HTTPS compression; TCP offloading; TCP buffering; direct server return; health checking; HTTP/HTTPS caching; content filtering; HTTP/HTTPS security; priority queuing; rate shaping; content-aware switching; client authentication; programmatic traffic manipulation; firewall; intrusion prevention systems.

Web server(s) 210 may include hardware (e.g., one or more computers) and/or software (e.g., one or more applications) that deliver web content that can be accessed by, for example a client device (e.g., user device 202) through a network (e.g., network 204), such as the Internet. Web server(s) 210 may use, for example, a hypertext transfer protocol (HTTP/HTTPS or sHTTP) to communicate with user device 202. The web pages delivered to client device may include, for example, HTML documents, which may include images, style sheets and scripts in addition to text content.

A user agent, such as, for example, a web browser, web crawler, or native mobile application, may initiate communication by making a request for a specific resource using HTTP/HTTPS and web server 210 may respond with the content of that resource or an error message if unable to do so. The resource may be, for example a file on stored on backend 218. Web server(s) 210 also may enable or facilitate receiving content from cardholder device 202 so cardholder device 202 may be able to, for example, submit web forms, including uploading of files.

Web server(s) also may support server-side scripting using, for example, Active Server Pages (ASP), PHP, or other scripting languages. Accordingly, the behavior of web server(s) 210 can be scripted in separate files, while the actual server software remains unchanged.

Load balancers 214 may be similar to load balancers 208 as described above.

Application server(s) 216 may include hardware and/or software that is dedicated to the efficient execution of procedures (e.g., programs, routines, scripts) for supporting its applied applications. Application server(s) 216 may comprise one or more application server frameworks, including, for example, Java application servers (e.g., Java platform, Enterprise Edition (Java EE), the .NET framework from Microsoft®, PHP application servers, and the like). The various application server frameworks may contain a comprehensive service layer model. Also, application server(s) 216 may act as a set of components accessible to, for example, an entity implementing system 200, through an API defined by the platform itself. For Web applications, these components may be performed in, for example, the same running environment as web server(s) 210, and application servers 216 may support the construction of dynamic pages. Application server(s) 216 also may implement services, such as, for example, clustering, fail-over, and load-balancing. In various embodiments, where application server(s) 216 are Java application servers, the web server(s) 216 may behaves like an extended virtual machine for running applications, transparently handling connections to databases associated with backend 218 on one side, and, connections to the Web client (e.g., client device 202) on the other.

Backend 218 may include hardware and/or software that enables the backend services of, for example, a medical facility and/or medical provider or other entity that maintains a distributed system similar to system 200. Backend 218 may be associated with various databases, including databases that maintain, for example, medical information (e.g., patient data, vital signs data, classifier data, and/or the like). Backend 218 also may be associated with one or more servers that enable the various services provided by system 200. Backend 218 may enable a medical facility and/or medical provider to implement various functions as shown and described herein.

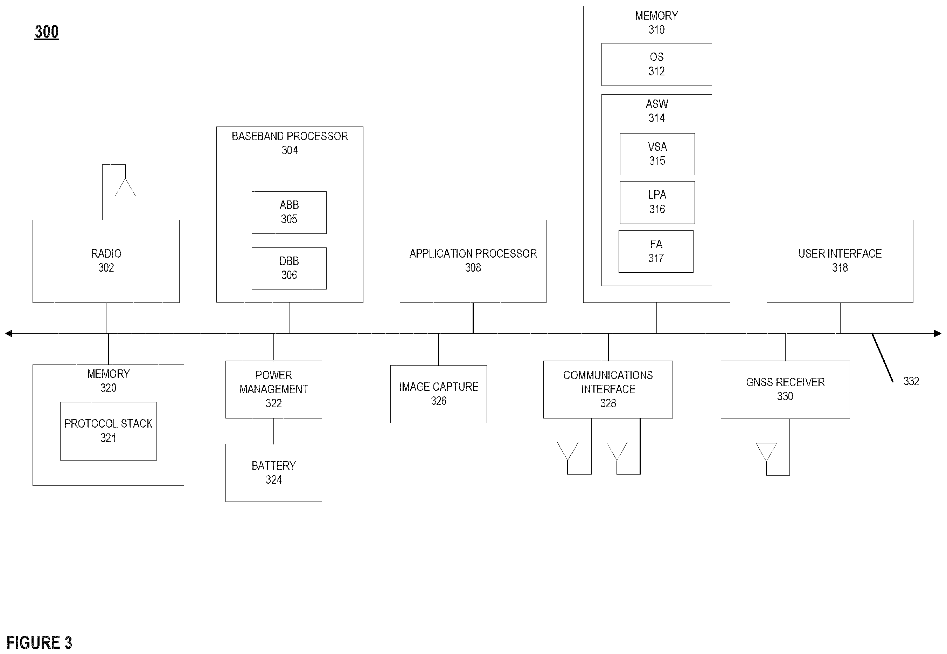

With reference to Figure 3, a monitoring system and/or mobile device 300, which may be similar (in whole or in part) to monitoring system 140 and/or mobile device 150, may be provided. For example, Figure 3 illustrates an example architecture (e.g., hardware and software) for an example mobile device and/or monitoring system 300. It should be appreciated by one having ordinary skill in the art in the context of the present disclosure that the architecture of the mobile device and/or monitoring system 300 depicted in Figure 3 is but one example, and that in some embodiments, additional, fewer, and/or different components may be used to achieve similar and/or additional functionality. Mobile device and/or monitoring system 300 may be embodied as a smartphone, a tablet, a mobile computing device, a patient monitoring system, and/or the like. And, in some embodiments, other types of devices may be used, including a workstation, laptop, notebook, etc. The mobile device and/or monitoring system 300 may be used in some embodiments to receive data from a wearable device (e.g., wearable device 120), further process data received from wearable device (e.g., wearable device 120), generate alerts and/or reporting data based on received data from wearable device (e.g., wearable device 120), and/or transmit wearable device data and/or alerts/reports based on wearable device data.

Mobile device and/or monitoring system 300 may include at least two different processors, such as a baseband processor (BBP) 304 and an application processor (APP) 308. A baseband processor 304 may primarily handle baseband communication-related tasks. An application processor 308 may handle inputs and outputs and/or all applications other than those directly related to baseband processing. The baseband processor 304 may include a dedicated processor for deploying functionality associated with a protocol stack, such as, but not limited to a GSM (Global System for Mobile communications) protocol stack, among other functions. The application processor 308 may include a multi-core processor for running applications, including all or a portion of application software. The baseband processor 304 and the application processor 308 may have respective associated memory including random access memory (RAM), Flash memory, etc., and peripherals, and a running clock. The memory may be each also referred to herein as a non-transitory computer readable medium. Note that, though depicted as residing in memory 310, all or a portion of the application software may be stored in memory 310, distributed among memory or reside in other memory.

The baseband processor 304 may deploy functionality of the protocol stack to enable the mobile device and/or monitoring system 300 to access one or a plurality of wireless network technologies, including WCDMA (Wideband Code Division Multiple Access), CDMA (Code Division Multiple Access), EDGE (Enhanced Data Rates for GSM Evolution), GPRS (General Packet Radio Service), Zigbee (e.g., based on IEEE 802.15.4), Bluetooth, Wi-Fi (Wireless Fidelity, such as based on IEEE 802.11), and/or LTE (Long Term Evolution), among variations thereof and/or other telecommunication protocols, standards, and/or specifications. The baseband processor 304 may manage radio communications and control functions, including signal modulation, radio frequency shifting, and encoding. The baseband processor 304 may include, or may be coupled to, a radio (e.g., RF front end) 302 and/or a GSM (or other communications standard) modem, and analog and digital baseband circuitry (ABB, DBB, receptively). The radio 302 may include one or more antennas, a transceiver, and a power amplifier to enable the receiving and transmitting of signals of a plurality of different frequencies, enabling access to a cellular (and/or wireless) network. The analog baseband circuitry may be coupled to the radio 302 and provide an interface between the analog and digital domains of the GSM modem. The analog baseband circuitry may include circuitry including an analog-to-digital converter (ADC) and digital-to-analog converter (DAC), as well as control and power management/distribution components and an audio codec to process analog and/or digital signals received indirectly via the application processor 308 or directly from a user interface (UI) 318 (e.g., microphone, earpiece, alert tone, vibrator circuits, touch-screen, etc.). The ADC may digitize any analog signals for processing by the digital baseband circuitry.

The digital baseband circuitry may deploy the functionality of one or more levels of the GSM protocol stack (e.g., Layer 1, Layer 2, etc.), and may include a microcontroller (e.g., microcontroller unit or MCU, also referred to herein as a processor) and a digital signal processor (DSP, also referred to herein as a processor) that communicate over a shared memory interface (the memory comprising data and control information and parameters that instruct the actions to be taken on the data processed by the application processor 308). The MCU may be embodied as a RISC (reduced instruction set computer) machine that runs a real-time operating system (RTIOS), with cores having a plurality of peripherals (e.g., circuitry packaged as integrated circuits) such as RTC (real-time clock), SPI (serial peripheral interface), I2C (inter-integrated circuit), UARTs (Universal Asynchronous Receiver/Transmitter), devices based on IrDA (Infrared Data Association), SD/MMC (Secure Digital/Multimedia Cards) card controller, keypad scan controller, and USB devices, GPRS crypto module, TDMA (Time Division Multiple Access), smart card reader interface (e.g., for the one or more SIM (Subscriber Identity Module) cards), timers, and among others. For receive-side functionality, the MCU may instruct the DSP to receive, for instance, in-phase/quadrature (I/Q) samples from the analog baseband circuitry and perform detection, demodulation, and decoding with reporting back to the MCU. For transmit-side functionality, the MCU presents transmittable data and auxiliary information to the DSP, which encodes the data and provides to the analog baseband circuitry (e.g., converted to analog signals by the DAC).

The application processor 308 may operate under control of an operating system (OS) that enables the implementation of a plurality of user applications, including, for example, the application software that supports vital sign calculations, alerting, and/or reporting. The application processor 308 may be embodied as a System on a Chip (SOC), and supports a plurality of multimedia related features including Internet/cloud-based access functionality to access one or more computing devices, of the cloud(s), that are coupled to the Internet. For instance, the application processor 308 may execute communications functionality of the application software (e.g., middleware which may include a browser with or operable in association with one or more application program interfaces (APIs)) to enable access to a cloud computing framework or other networks to provide remote data access/storage/processing, and through cooperation with an embedded operating system, access to calendars, location services, user data, public data, vital signs data, contact data, etc.

For instance, in some embodiments, vital signs calculations, alerting, and/or reporting may operate using cloud computing services, where the processing of raw and/or derived parameter data received, indirectly at mobile device and/or monitoring system 300 or directly from wearable device (e.g., wearable device 120) may be achieved by one or more devices of the cloud(s), and triggering signals (to trigger feedback) may be communicated from the cloud(s) (or other devices) to the mobile device and/or monitoring system 300, which in turn may activate feedback internal to mobile device and/or monitoring system 300 or relay the triggering signals to other devices.

The application processor 308 may include a processor core (Advanced RISC Machine or ARM), and further comprises or may be coupled to multimedia modules (for decoding/encoding pictures, video, and/or audio), a graphics processing unit (GPU), communications interface 328, and device interfaces. Communications interfaces 328 may include wireless interfaces, including a Bluetooth (BT) (and/or Zigbee in some embodiments, among others) module that enable wireless communication with any system component.

Communication interface 328 may include a Wi-Fi module for interfacing with a local 802.11 network, according to corresponding communications software in the applications software. The application processor 308 may include or be coupled to, a global navigation satellite systems (GNSS) receiver 330 for enabling access to a satellite network to, for instance, provide position coordinates. In some embodiments, the GNSS receiver 330, in association with GNSS functionality in the application software, may collect contextual data (time and location data, including location coordinates and altitude) to determine location data associated with the mobile device and/or monitoring system 300. Note that, though described as a GNSS receiver 330, other indoor/outdoor positioning systems may be used, including those based on triangulation of cellular network signals and/or Wi-Fi.

The device interfaces coupled to the application processor 308 may include the user interface 318, such as a display screen. The display screen may be embodied in one of several available technologies, including LCD or Liquid Crystal Display (or variants thereof, such as Thin Film Transistor (TFT) LCD, In Plane Switching (IPS) LCD)), light-emitting diode (LED)-based technology, such as organic LED (OLED), Active-Matrix OLED (AMOLED), retina or haptic-based technology, or virtual/augmented reality technology. For instance, the user interface 318 may present visual feedback in the form of messaging (e.g., alphanumerical) and/or symbols/graphics (e.g., warning or alert icons, flashing screen, etc.), and/or flashing lights (LEDs). In some embodiments, the user interface 318 may be configured, in addition to or in lieu of a display screen, a keypad, microphone, speaker, ear piece connector, I/O interfaces (e.g., USB (Universal Serial Bus)), SD/MMC card, among other peripherals. For instance, the speaker may be used to audibly provide feedback, and/or the user interface 318 may comprise a vibratory motor that provides a vibrating feedback to the user. One or any combination of visual, audible, or tactile feedback may be used, and as described before, variations in the intensity of format of the feedback may be used.

Also coupled to the application processor 308 may be an image capture device (IMAGE CAPTURE) 326. The image capture device 326 may include an optical sensor (e.g., a charged coupled device (CCD) or a complementary metal-oxide semiconductor (CMOS) optical sensor). The image capture device 326 may be configured to capture and/or receive medical imaging, such as ultrasounds and/or other scans.

A power management device 322 may control and manages operations of a battery 324 and/or other power supplies. The components described above and/or depicted in Figure 3 may share data over one or more busses, such as for example via data bus 332. It should be appreciated by one having ordinary skill in the art, in the context of the present disclosure, that variations to the above may be deployed in some embodiments to achieve similar functionality.

An application processor 308 may run and/or execute application software 314, and may include a vital signs application 315, a language processing application 316, and/or a feedback application 317.

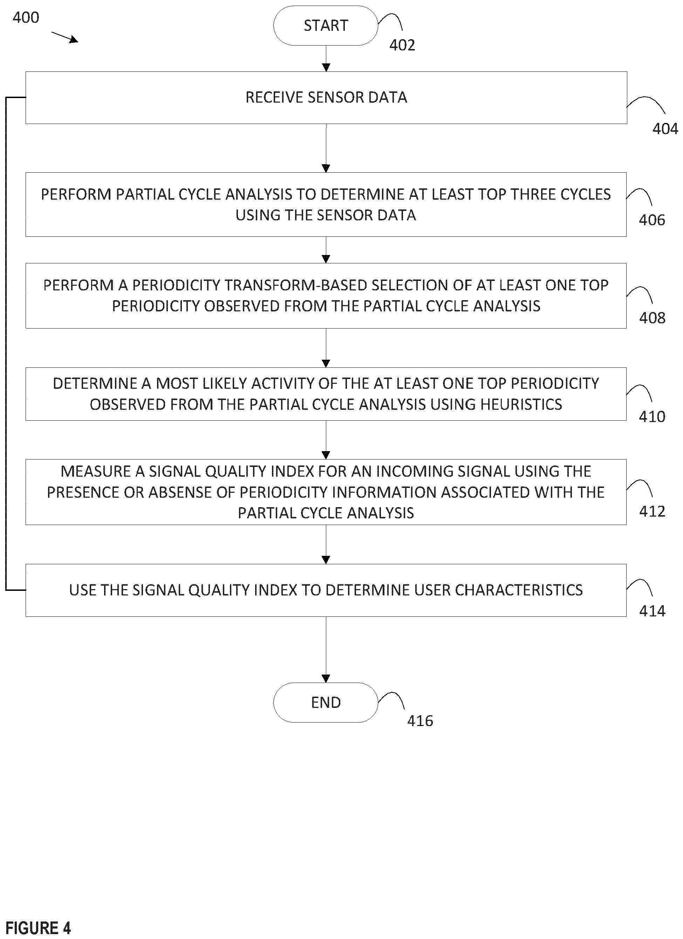

A method that utilizes the system components described herein is described with reference to Figure 4. For example, a method may begin at block 402. At block 404, a wearable device, such as a wearable deice 120, may receive raw sensor data via one or more sensors on the wearable device, such as accelerometer data, gyroscope data, magnetometer data, barometer data, PPG data, ECG data, and/or the like.

At block 406, an initial processing may take place and a partial cycle analysis may determine at least the top three cycles at any point in time. By way of example, an initial processing may include smoothing and down-sample the raw sensor data (e.g., input signals or the time series of measurements). A partial cycle analysis may include locating the fractional cycle markers (e.g., waveform peaks - one to three scales, waveform valleys - one to three scales, and/or zero crossing sections). A partial cycle analysis may include approximating the cycle lengths and amplitudes for at least three cycles (e.g., A1, CL1, A2, CL2, A3, CL3). A partial cycle analysis may assume three maximum periodicities in a body-worn sensor. A partial cycle analysis may approximate the expected periodicities (e.g., P1, P2, P3) and relate them to an underlying mathematical model.

An underlying mathematical model for chest worn sensor may be presented as:

$$\{y(n)\}=\{x_r(n)\}+\{x_g(n)\}+\{x_a(n)\}+\{w(n)\} \qquad (1)$$

where

$\{y(n)\}$ = Observed chest sensor signal
$\{x_r(n)\}$ = measured respiration signal
$\{x_g(n)\}$ -measured gait signal
$\{x_a(n)\}$ = measured artifact signal and
$\{w(n)\}$ = white noise signal

At block 408, a system component, e.g., a wearable device and/or an implantable device and/or a device in communication with a wearable and/or implantable device, may perform a periodicity transform-based selection of at least one top periodicity observed from the respiratory cycle analysis. Using a periodicity transform-based selection, digital signals and/or time series of physiological data may be detected. For example, a periodicity transform may decompose a sequence into a sum of periodic sequences by projecting onto a set of periodic subspaces, Pp, which may result in leaving residuals where periodicities have been removed. A resulting representation of the data may then be linear-in-period rather than linear-in-frequency or linear-in-scale.

By way of example, a periodicity-transform-based selection may include estimating the most likely cycle length based on amplitudes (e.g., A1, A2, A3) and the available cycle lengths (e.g., CL1, CL2, CL3). In this manner, a periodicity-transform-based selection may include determining whether adjacent periodicities show dominant characteristics due to the variability inside the measurement interval and, if so, determining that periodicity bands are formed and nominating a center periodicity inside the band as the most likely cycle length.

By way of example, where there are three expected periodicities, a periodicity may be set to 1 (e.g., P1 = 1). A linear DC component may then be removed by removing the projection onto the periodicity (e.g., P1 = 1). Next, a periodicity may be set to a cycle length (e.g., P1 = CL1) and a device may check to see if a projection (e.g., A1) includes at least a threshold (e.g., T) percentage of the energy in a signal (e.g., X). If so, a device may accept the projection (e.g., A1) and cycle length (e.g., CL1) as a possible cycle that may be present. This may be repeated for each identified cycle (e.g., CL2, CL3). A most likely cycle length may then be estimated based on the available amplitudes and cycle lengths using the determine data, variability inside the measurement interval, and periodicity bands.

Additionally, a max power cycle length for a filtered time series of sensor data, a mean power cycle length for a filtered time serious of sensor data, and a maximum significant estimated cycle length for a filtered time series of data may be used to compute the most likely activity for a particular time.

At block 410, a system component, e.g., a wearable device and/or an implantable device and/or a device in communication with a wearable and/or implantable device, may determine a most likely activity of the at least one top periodicity observed from the partial cycle analysis using heuristics. Heuristics may include data and/or data features regarding characteristics of a respiratory signal, characteristics of a gait signal, characteristics of a motion artifact signal, and/or characteristics of baseline electronic noise. By way of example, data and/or data features that may represent characteristics of a respiratory signal may include, continuity, a particular amplitude range, one or more particular cycle lengths (periodicities), a pseudo-periodic nature, a dominance in a few channels of sensors (if not in all), and a correlation with measurements made in silent intervals. By way of example, data and/or data features that may represent characteristics of a gait signal may include, a continuity for a short duration of time, a particular amplitude range, one or more particular cycle length(s) (periodicities), a pseudo-periodic nature, and a difference from measurements made in silent intervals. By way of example, data and/or data features that may represent characteristics of a motion artifact signal may include, a lack of continuity, a particular amplitude range, one or more particular cycle lengths (periodicities) (aperiodic), a dominance in a few channels of sensors (if not all), and a non-correlation with measurements made in silent intervals. By way of example, data and/or data features that may represent characteristics of baseline electronic noise may include: a lack of periodicity of any time, a non-correlation between segments, and bounded amplitudes.

At block 412, a system component, e.g., a wearable device and/or an implantable device and/or a device in communication with a wearable and/or implantable device, may measure a signal quality index for an incoming signal using the presence or absence of periodicity information associated with the partial cycle analysis. By way of example, the data features present in the heuristics may be used to determine a signal quality index. Or rather, the data features in each activity band may be defined by information continuity, band-limitedness, and/or the absence of presence of noise. A signal quality index may identify non-significance as defined by sampling content outside a region of interest in time and/or frequency domains. By way of example, activities such as ambulation and/or climbing stairs may induce higher frequency content and may be outside the band of respiratory activity (e.g., episode 1). Similarly, complete inactivity due to lack of sensor adhesion and/or contact may result in DC content (e.g., episode 2). Both of these types of episodes may lie outside the region of interest.

At block 414, a system component, e.g., a wearable device and/or an implantable device and/or a device in communication with a wearable and/or implantable device, may use the signal quality index to determine user characteristics and/or device status information. For example, a sudden change in a signal quality index may indicate a possible posture change. As another example, a sudden increase in DC may push the signal quality index down and will indicate device removal and/or sensor error(s).

The method may end at block 416.

These examples are merely illustrative and transaction cards may be reprogrammed according to any data described herein.

It is further noted that the systems and methods described herein may be tangibly embodied in one of more physical media, such as, but not limited to, a compact disc (CD), a digital versatile disc (DVD), a floppy disk, a hard drive, read only memory (ROM), random access memory (RAM), as well as other physical media capable of storing software, or combinations thereof. Moreover, the figures illustrate various components (e.g., servers, computers, processors, etc.) separately. The functions described as being performed at various components may be performed at other components, and the various components bay be combined or separated. Other modifications also may be made.

The present disclosure is not to be limited in terms of the particular embodiments described in this application, which are intended as illustrations of various aspects. Many modifications and variations can be made without departing from its spirit and scope, as may be apparent. Functionally equivalent methods and apparatuses within the scope of the disclosure, in addition to those enumerated herein, may be apparent from the foregoing representative descriptions. Such modifications and variations are intended to fall within the scope of the appended representative claims. The present disclosure is to be limited only by the terms of the appended representative claims, along with the full scope of equivalents to which such representative claims are entitled. It is also to be understood that the terminology used herein is for the purpose of describing particular embodiments only, and is not intended to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It may be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It may be further understood by those within the art that if a specific number of an introduced claim recitation is intended, such an intent may be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It may be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" may be understood to include the possibilities of "A" or "B" or "A and B."

The foregoing description, along with its associated embodiments, has been presented for purposes of illustration only. It is not exhaustive and does not limit the invention to the precise form disclosed. Those skilled in the art may appreciate from the foregoing description that modifications and variations are possible in light of the above

EP 3 958 737 B1

teachings or may be acquired from practicing the disclosed embodiments. For example, the steps described need not be performed in the same sequence discussed or with the same degree of separation. Likewise various steps may be omitted, repeated, or combined, as necessary, to achieve the same or similar objectives. Accordingly, the invention is not limited to the above-described embodiments, but instead is defined by the appended claims in light of their full scope of equivalents.

In the preceding specification, various preferred embodiments have been described with references to the accompanying drawings. It may, however, be evident that various modifications and changes may be made thereto, and additional embodiments may be implemented, without departing from the broader scope of the invention as set forth in the claims that follow. The specification and drawings are accordingly to be regarded as an illustrative rather than restrictive sense.

**Claims**

1. A device (120) comprising:

    an accelerometer (122) configured to receive accelerometer data;
    a gyroscope (123) configured to receive gyroscope data; and
    a microprocessor (129) configured to:

        perform partial cycle analysis to determine at least a top three cycles based on at least one of: the accelerometer data and gyroscope data, wherein the partial cycle analysis comprises:

            identifying in the at least one of the accelerometer data or gyroscope data one or more fractional cycle markers, the fractional cycle markers including at least one of: waveform peaks, waveform valleys and zero crossings;
            determining the at least top three cycles based on approximating cycle lengths, CL, and amplitudes, A, of at least three cycles assumed present in the data based on the fractional cycle markers; and
            approximating an expected periodicity, P, of each of the three cycles based on the cycle lengths, CL, for each of the three cycles;

        perform a periodicity transform (PT)-based selection of at least one top periodicity observed from the partial cycle analysis, the PT-based selection comprising, for each of the expected periodicities, P, of the three cycles:

            projecting the data onto a periodicity subspace for the respective expected periodicity, P;
            checking if the projection includes at least a threshold, T, percentage of a total energy in a signal comprised by the data, and if so accepting the cycle to which the periodicity corresponds as a possible cycle present in the data; and
            estimating a most likely cycle length based on the cycle lengths and amplitudes of the identified possible cycles; and

        determine a most likely activity of the at least one top periodicity using heuristics.

2. The device of claim 1, wherein the PT-based selection is performed by:

    (a) assuming a threshold (T);
    (b) obtaining signal X of length N via at least one of: the accelerometer data and the gyroscope data;
    (c) locating a fractional cycle marker for each cycle of the at least top three cycles;
    (d) approximating a cycle length ($CL_i$) and an amplitude ($A_i$) for each cycle, wherein i is a cycle;
    (e) approximating the expected periodicities for each cycle;
    (f) relating the expected periodicities to the fractional cycle marker for each cycle;
    (g) letting an expected periodicity equal to one;
    (h) removing the linear DC component by removing the projection onto the first expected periodicity equal to one;
    (i) letting the expected periodicity equal $CL_1$;
    (j) determining whether $A_1$ contains at least T percentage of energy in X;
    (k) if $A_1$ contains at least T percentage of energy in X, accepting $A_1$ and $CL_1$ as a possible cycle;
    (l) repeating steps (i)-(k) for each $CL_i$ and $A_i$; and
    (m) estimating a most likely cycle length based on each cycle length ($CL_i$) and an amplitude ($A_i$) for each cycle.

3. The device of claim 2, wherein if adjacent periodicities show dominant characteristics due to a variability inside the measurement interval, periodicity bands are formed and a center periodicity inside a band is nominated as the most likely cycle length.

4. The device of claim 1, wherein heuristics is based on at least one of: a particular amplitude range, a particular cycle length, data indicative of continuity, data indicative of a duration of continuity, data indicative of a periodic nature, data indicative of what channels of sensors a signal is dominant in, and a degree of correlation with measurements made in silent intervals.

5. The device of claim 1, wherein the microprocessor is further configured to measure a signal quality index (SQI) for an incoming signal associated with at least one of the accelerometer data and gyroscope data using the presence or absence of periodicity information associated with the partial cycle analysis, and optionally wherein sudden changes in SQI for a duration less than a first SQI threshold indicate a posture change.

6. The device of claim 1, wherein the accelerometer and gyroscope are housed in a wearable device, and optionally wherein the wearable device is configured to be worn on a patient's chest.

7. The device of claim 1, wherein the accelerometer data and/or the gyroscope data is received on-demand.

8. The device of claim 1, wherein the microprocessor is further configured to determine a signal quality index (SQI) associated with the most likely activity.

9. The device of claim 1, wherein the heuristics comprise at least one of: a maximum power cycle length associated with at the accelerometer data, a maximum power cycle length associated with the gyroscope data, a mean power cycle length associated with at the accelerometer data, a mean power cycle length associated with the gyroscope data, a maximum significant estimated cycle length associated with at the accelerometer data, a maximum significant estimated cycle length associated with the gyroscope data.

10. The device of claim 1, wherein the partial cycle analysis is performed by continuously mapping cycles onto regions of maximum amplitudes, minimum amplitudes, and zero crossing intervals and their periodicities.

11. The device of claim 10, wherein a cycle is selected as an at least top three cycles based on:

   amplitude thresholds and repeatability of periodicity; and/or
   a valid signal quality index.

12. The device of claim 10, wherein a baseline measurement noise amplitude is computed and subtracted from amplitude measurements associated with at least one of accelerometer data and gyroscope data.

13. A method comprising:

   receiving accelerometer data (404) via an accelerometer of a wearable device;
   receiving gyroscope data (404) via a gyroscope of the wearable device;
   performing partial cycle analysis (406) to determine at least a top three cycles based on at least one of: the accelerometer data and gyroscope data, wherein the partial cycle analysis comprises:

      identifying in the at least one of the accelerometer data or gyroscope data one or more fractional cycle markers, the fractional cycle markers including at least one of: waveform peaks, waveform valleys and zero crossings;
      determining the at least top three cycles based on approximating cycle lengths, CL, and amplitudes, A, of at least three cycles assumed present in the data based on the fractional cycle markers; and
      approximating an expected periodicity, P, of each of the three cycles based on the cycle lengths, CL, for each of the three cycles;

   performing a periodicity transform (PT)-based selection of at least one top periodicity observed from the partial cycle analysis (408), the PT-based selection comprising, for each of the expected periodicities, P, of the three cycles:

projecting the data onto a periodicity subspace for the respective expected periodicity, P;

checking if the projection includes at least a threshold, T, percentage of a total energy in a signal comprised by the data, and if so accepting the cycle to which the periodicity corresponds as a possible cycle present in the data; and

estimating a most likely cycle length based on the cycle lengths and amplitudes of the identified possible cycles; and

determining a most likely activity of the at least one top periodicity using heuristics (410).

**14.** The method of claim 13, wherein

the most likely activity is respiration and the top periodicity is a respiration rate; and/or
wherein the most likely activity comprises at least one of: sudden motion, sustained motion, slow chest wall movement, deep chest wall movement, and vigorous vibration.

**15.** The method of claim 13, wherein:

the ratios of amplitudes of most likely cycle length indicate a signal quality index; and/or
the amplitudes of most likely cycle lengths and a presence of each amplitude in relation to a region of interest determine a signal quality index for each estimation.

**Patentansprüche**

**1.** Vorrichtung (120), umfassend:

einen Beschleunigungsmesser (122), der konfiguriert ist, um Beschleunigungsmesserdaten zu empfangen;
ein Gyroskop (123), das konfiguriert ist, um Gyroskopdaten zu empfangen; und
einen Mikroprozessor (129), der konfiguriert ist, um:

eine partielle Zyklusanalyse durchzuführen, um mindestens drei wichtigste Zyklen basierend auf mindestens einem zu bestimmen von: den Beschleunigungsmesserdaten und Gyroskopdaten, wobei die partielle Zyklusanalyse umfasst:

Identifizieren von einer oder mehreren Teilzyklusmarkierungen in mindestens einem der Beschleunigungsmesserdaten oder Gyroskopdaten, wobei die Teilzyklusmarkierungen mindestens eines beinhalten von: Wellenformspitzen, Wellenformtälern und Nulldurchgängen;
Bestimmen der mindestens drei wichtigsten Zyklen basierend auf Annähern von Zykluslängen, CL, und Amplituden, A, von mindestens drei Zyklen, die als in den Daten vorhanden angenommen werden, basierend auf den Teilzyklusmarkierungen; und
Annähern einer erwarteten Periodizität, P, jedes der drei Zyklen basierend auf den Zykluslängen, CL, für jeden der drei Zyklen;

eine Periodizitätstransformations-, (PT)-basierte Auswahl von mindestens einer Top-Periodizität durchzuführen, die aus der partiellen Zyklusanalyse beobachtet wird, wobei die PT-basierte Auswahl für jede der erwarteten Periodizitäten, P, der drei Zyklen umfasst:

Projizieren der Daten auf einen Periodizitäts-Teilraum für die jeweilige erwartete Periodizität, P;
Überprüfen, ob die Projektion mindestens einen Schwellenwert-, T-, Prozentsatz einer Gesamtenergie in einem zu den Daten gehörenden Signal beinhaltet, und, wenn dies der Fall ist, Akzeptieren des Zyklus, dem die Periodizität entspricht, als möglichen in den Daten vorhandenen Zyklus; und
Schätzen einer wahrscheinlichsten Zykluslänge basierend auf den Zykluslängen und Amplituden der identifizierten möglichen Zyklen; und

eine wahrscheinlichste Aktivität der mindestens einen Top-Periodizität unter Verwendung von Heuristik zu bestimmen.

**2.** Vorrichtung nach Anspruch 1, wobei die PT-basierte Auswahl durchgeführt wird durch:

(a) Annehmen eines Schwellenwertes (T);

(b) Erhalten eines Signals X der Länge N aus mindestens einem von: den Beschleunigungsmesserdaten und den Gyroskopdaten;

(c) Lokalisieren einer Teilzyklusmarkierung für jeden Zyklus der mindestens drei wichtigsten Zyklen;

(d) Annähern einer Zykluslänge ($CL_i$) und einer Amplitude ($A_i$) für jeden Zyklus, wobei i ein Zyklus ist;

(e) Annähern der erwarteten Periodizitäten für jeden Zyklus;

(f) Setzen der erwarteten Periodizitäten in Beziehung zu der Teilzyklusmarkierung für jeden Zyklus;

(g) Belassen einer erwarteten Periodizität bei gleich eins;

(h) Entfernen der linearen Gleichstromkomponente durch Entfernen der Projektion auf die erste erwartete Periodizität gleich eins;

(i) Belassen der erwarteten Periodizität bei gleich $CL_1$;

(j) Bestimmen, ob $A_1$ mindestens einen T-Prozentsatz an Energie in X enthält;

(k) wenn $A_1$ mindestens einen T-Prozentsatz an Energie in X enthält, Akzeptieren von $A_1$ und $CL_1$ als möglichen Zyklus;

(l) Wiederholen der Schritte (i)-(k) für jede $CL_i$ und $A_i$; und

(m) Schätzen einer wahrscheinlichsten Zykluslänge basierend auf jeder Zykluslänge ($CL_i$) und einer Amplitude ($A_i$) für jeden Zyklus.

3. Vorrichtung nach Anspruch 2, wobei, wenn benachbarte Periodizitäten aufgrund einer Variabilität innerhalb des Messintervalls dominante Charakteristiken aufzeigen, Periodizitätsbänder gebildet werden und eine zentrale Periodizität innerhalb eines Bandes als die wahrscheinlichste Zykluslänge nominiert wird.

4. Vorrichtung nach Anspruch 1, wobei Heuristik auf mindestens einem basiert von:

einem bestimmten Amplitudenbereich, einer bestimmten Zykluslänge, Daten, die bezeichnend für Kontinuität sind, Daten, die bezeichnend für eine Dauer der Kontinuität sind, Daten, die bezeichnend für eine periodische Natur sind, Daten, die bezeichnend dafür sind, in welchen Sensorkanälen ein Signal dominant ist, und einem Grad der Korrelation mit Messungen, die in stillen Intervallen durchgeführt werden.

5. Vorrichtung nach Anspruch 1, wobei der Mikroprozessor weiter konfiguriert ist, um einen Signalqualitätsindex (SQI) für ein eingehendes Signal, das mindestens einem der Beschleunigungsmesserdaten und Gyroskopdaten zugeordnet ist, unter Verwendung des Vorhandenseins oder Fehlens von Periodizitätsinformationen zu messen, die der partiellen Zyklusanalyse zugeordnet sind, und wobei optional plötzliche Änderungen im SQI für eine Dauer von weniger als einem ersten SQI-Schwellenwert eine Haltungsänderung angeben.

6. Vorrichtung nach Anspruch 1, wobei der Beschleunigungsmesser und das Gyroskop in einer tragbaren Vorrichtung aufgenommen sind, und wobei die tragbare Vorrichtung optional konfiguriert ist, um auf der Brust eines Patienten getragen zu werden.

7. Vorrichtung nach Anspruch 1, wobei die Beschleunigungsmesserdaten und/oder die Gyroskopdaten auf Anfrage empfangen werden.

8. Vorrichtung nach Anspruch 1, wobei der Mikroprozessor weiter konfiguriert ist, um einen Signalqualitätsindex (SQI) zu bestimmen, welcher der wahrscheinlichsten Aktivität zugeordnet ist.

9. Vorrichtung nach Anspruch 1, wobei die Heuristik mindestens eines umfasst von: einer maximalen Leistungszykluslänge, die den Beschleunigungsmesserdaten zugeordnet ist, einer maximale Leistungszykluslänge, die den Gyroskopdaten zugeordnet ist, einer mittleren Leistungszykluslänge, die den Beschleunigungsmesserdaten zugeordnet ist, einer mittleren Leistungszykluslänge, die den Gyroskopdaten zugeordnet ist, einer maximalen signifikanten geschätzten Zykluslänge, die den Beschleunigungsmesserdaten zugeordnet ist, einer maximalen signifikanten geschätzten Zykluslänge, die den Gyroskopdaten zugeordnet ist.

10. Vorrichtung nach Anspruch 1, wobei die partielle Zyklusanalyse durch kontinuierliches Abbilden von Zyklen auf Bereiche mit maximalen Amplituden, minimalen Amplituden und Nulldurchgangsintervallen und deren Periodizitäten durchgeführt wird.

11. Vorrichtung nach Anspruch 10, wobei ein Zyklus als einer von mindestens drei wichtigsten Zyklen ausgewählt wird, basierend auf:

Amplitudenschwellenwerten und Wiederholbarkeit der Periodizität; und/oder einem gültigen Signalqualitätsindex.

**12.** Vorrichtung nach Anspruch 10, wobei eine Basismessrauschamplitude berechnet und von Amplitudenmessungen subtrahiert wird, die mindestens einem der Beschleunigungsmesserdaten und Gyroskopdaten zugeordnet sind.

**13.** Verfahren, umfassend:

Empfangen von Beschleunigungsmesserdaten (404) über einen Beschleunigungsmesser einer tragbaren Vorrichtung;
Empfangen von Gyroskopdaten (404) über ein Gyroskop der tragbaren Vorrichtung;
Durchführen einer partiellen Zyklusanalyse (406), um mindestens drei wichtigste Zyklen basierend auf mindestens einem zu bestimmen von: den Beschleunigungsmesserdaten und Gyroskopdaten, wobei die partielle Zyklusanalyse umfasst:

Identifizieren von einer oder mehreren Teilzyklusmarkierungen in mindestens einem der Beschleunigungsmesserdaten oder Gyroskopdaten, wobei die Teilzyklusmarkierungen mindestens eines beinhalten von: Wellenformspitzen, Wellenformtälern und Nulldurchgängen;
Bestimmen der mindestens drei wichtigsten Zyklen basierend auf Annähern von Zykluslängen, CL, und Amplituden, A, von mindestens drei Zyklen, die als in den Daten vorhanden angenommen werden, basierend auf den Teilzyklusmarkierungen; und
Annähern einer erwarteten Periodizität, P, jedes der drei Zyklen basierend auf den Zykluslängen, CL, für jeden der drei Zyklen;

Durchführen einer Periodizitätstransformations-, (PT)-basierten Auswahl von mindestens einer Top-Periodizität, die aus der partiellen Zyklusanalyse (408) beobachtet wird, wobei die PT-basierte Auswahl für jede der erwarteten Periodizitäten, P, der drei Zyklen umfasst:

Projizieren der Daten auf einen Periodizitäts-Teilraum für die jeweilige erwartete Periodizität, P;
Überprüfen, ob die Projektion mindestens einen Schwellenwert-, T-, Prozentsatz einer Gesamtenergie in einem zu den Daten gehörenden Signal beinhaltet, und, wenn dies der Fall ist, Akzeptieren des Zyklus, dem die Periodizität entspricht, als möglichen in den Daten vorhandenen Zyklus; und
Schätzen einer wahrscheinlichsten Zykluslänge basierend auf den Zykluslängen und Amplituden der identifizierten möglichen Zyklen; und

Bestimmen einer wahrscheinlichsten Aktivität der mindestens einen Top-Periodizität unter Verwendung von Heuristik (410).

**14.** Verfahren nach Anspruch 13, wobei

die wahrscheinlichste Aktivität Atmung ist und die Top-Periodizität die Atemfrequenz ist; und/oder
wobei die wahrscheinlichste Aktivität mindestens eines umfasst von: plötzlicher Bewegung, anhaltender Bewegung, langsamer Bewegung der Brustwand, tiefer Bewegung der Brustwand und kräftiger Vibration.

**15.** Verfahren nach Anspruch 13, wobei:

die Verhältnisse von Amplituden der wahrscheinlichsten Zykluslänge einen Signalqualitätsindex angeben; und/oder
die Amplituden der wahrscheinlichsten Zykluslängen und ein Vorhandensein jeder Amplitude in Bezug auf einen Bereich von Interesse einen Signalqualitätsindex für jede Schätzung bestimmen.

**Revendications**

**1.** Dispositif (120), comprenant :

un accéléromètre (122) configuré pour recevoir des données d'accéléromètre ;
un gyroscope (123) configuré pour recevoir des données de gyroscope ; et
un microprocesseur (129) configuré pour :

effectuer une analyse de cycle partiel pour déterminer au moins trois premiers cycles sur la base d'au moins un élément parmi : les données d'accéléromètre et les données de gyroscope, dans lequel l'analyse de cycle partiel comprend :

l'identification, dans le au moins un élément parmi les données d'accéléromètre ou les données de gyroscope, d'un ou plusieurs marqueurs de cycles fractionnaires, les marqueurs de cycle fractionnaire incluant au moins un élément parmi : des pics de forme d'onde, des creux de forme d'onde et des passages par zéro ;

la détermination des au moins trois premiers cycles sur la base de longueurs de cycle, CL, et d'amplitudes, A, approximatives d'au moins trois cycles supposés présents dans les données sur la base des marqueurs de cycles fractionnaires ; et

le calcul approximatif d'une périodicité, P, attendue de chacun des trois cycles sur la base des longueurs de cycle, CL, pour chacun des trois cycles ;

effectuer une sélection basée sur la transformée de périodicité (PT) d'au moins une première périodicité observée à partir de l'analyse de cycle partiel, la sélection basée sur la PT comprenant, pour chacune des périodicités, P, attendues des trois cycles :

la projection des données sur un sous-espace de périodicité pour la périodicité, P, attendue respective ;

la vérification pour établir si la projection inclut au moins un pourcentage seuil, T, d'une énergie totale dans un signal constitué par les données, et si tel est le cas, l'acceptation du cycle auquel correspond la périodicité comme un cycle possible présent dans les données ; et

l'estimation d'une longueur de cycle la plus probable sur la base des longueurs de cycle et des amplitudes des cycles possibles identifiés ; et

déterminer l'activité la plus probable de la au moins une première périodicité à l'aide de l'heuristique.

2. Dispositif selon la revendication 1, dans lequel la sélection basée sur la PT est effectuée par :

(a) la supposition d'un seuil (T) ;

(b) l'obtention d'un signal X de longueur N par l'intermédiaire d'au moins un élément parmi : les données d'accéléromètre et les données de gyroscope ;

(c) la localisation d'un marqueur de cycle fractionnaire pour chaque cycle des au moins trois premiers cycles ;

(d) le calcul approximatif d'une longueur de cycle ($CL_i$) et d'une amplitude ($A_i$) pour chaque cycle, dans lequel i est un cycle ;

(e) le calcul approximatif des périodicités attendues pour chaque cycle ;

(f) la mise en relation des périodicités attendues avec le marqueur de cycle fractionnaire pour chaque cycle ;

(g) la conservation d'une périodicité attendue égale à un ;

(h) la suppression de la composante continue linéaire en supprimant la projection sur la première périodicité attendue égale à un ;

(i) la conservation de la périodicité attendue égale à $CL_1$ ;

(j) la détermination pour établir si $A_1$ contient au moins un pourcentage T d'énergie dans X ;

(k) si $A_1$ contient au moins un pourcentage T d'énergie dans X, l'acceptation d'$A_1$ et de $CL_1$ comme cycle possible ;

(l) la répétition des étapes (i)-(k) pour chaque $CL_i$ et $A_i$ ; et

(m) l'estimation d'une longueur de cycle la plus probable sur la base de chaque longueur de cycle ($CL_i$) et d'une amplitude ($A_i$) pour chaque cycle.

3. Dispositif selon la revendication 2, dans lequel, si des périodicités adjacentes présentent des caractéristiques dominantes en raison d'une variabilité à l'intérieur de l'intervalle de mesure, des bandes de périodicité sont formées et une périodicité centrale à l'intérieur d'une bande est désignée comme la longueur de cycle la plus probable.

4. Dispositif selon la revendication 1, dans lequel l'heuristique est basée sur au moins un élément parmi : une plage d'amplitude particulière, une longueur de cycle particulière, des données indiquant une continuité, des données indiquant une durée de continuité, des données indiquant une nature périodique, des données indiquant dans quels canaux de capteurs un signal est dominant, et un degré de corrélation avec des mesures effectuées dans des intervalles silencieux.

5. Dispositif selon la revendication 1, dans lequel le microprocesseur est en outre configuré pour mesurer un indice de qualité de signal (SQI) pour un signal entrant associé à au moins un élément parmi les données d'accéléromètre et les données de gyroscope en utilisant la présence ou l'absence d'informations de périodicité associées à l'analyse de cycle partiel, et éventuellement dans lequel des changements soudains de SQI pendant une durée inférieure à un premier seuil de SQI indiquent un changement de posture.

6. Dispositif selon la revendication 1, dans lequel l'accéléromètre et le gyroscope sont logés dans un dispositif portable, et éventuellement dans lequel le dispositif portable est configuré pour être porté sur la poitrine d'un patient.

7. Dispositif selon la revendication 1, dans lequel les données d'accéléromètre et/ou les données de gyroscope sont reçues à la demande.

8. Dispositif selon la revendication 1, dans lequel le microprocesseur est en outre configuré pour déterminer un indice de qualité de signal (SQI) associé à l'activité la plus probable.

9. Dispositif selon la revendication 1, dans lequel l'heuristique comprend au moins un élément parmi : une longueur de cycle de puissance maximale associée aux données d'accéléromètre, une longueur de cycle de puissance maximale associée aux données de gyroscope, une longueur de cycle de puissance moyenne associée aux données d'accéléromètre, une longueur de cycle de puissance moyenne associée aux données de gyroscope, une longueur de cycle estimée pertinente maximale associée aux données d'accéléromètre, une longueur de cycle estimée pertinente maximale associée aux données de gyroscope.

10. Dispositif selon la revendication 1, dans lequel l'analyse de cycle partiel est effectuée en mappant en continu des cycles sur des régions d'amplitudes maximales, d'amplitudes minimales et d'intervalles de passage par zéro et leurs périodicités.

11. Dispositif selon la revendication 10, dans lequel un cycle est sélectionné en tant qu'au moins trois premiers cycles sur la base de :

   seuils d'amplitude et de la répétabilité de la périodicité ; et/ou
   un indice de qualité de signal valide.

12. Dispositif selon la revendication 10, dans lequel une amplitude de bruit de mesure de base est calculée et soustraite des mesures d'amplitude associées à au moins un élément parmi les données d'accéléromètre et les données de gyroscope.

13. Procédé comprenant :

   la réception de données d'accéléromètre (404) par l'intermédiaire d'un accéléromètre d'un dispositif portable ;
   la réception de données de gyroscope (404) par l'intermédiaire d'un gyroscope du dispositif portable ;
   l'exécution d'une analyse de cycle partiel (406) pour déterminer au moins trois premiers cycles sur la base d'au moins un élément parmi : les données d'accéléromètre et les données de gyroscope, dans lequel l'analyse de cycle partiel comprend :

      l'identification, dans le au moins un élément parmi les données d'accéléromètre ou les données de gyroscope, d'un ou plusieurs marqueurs de cycles fractionnaires, les marqueurs de cycle fractionnaire incluant au moins un élément parmi : des pics de forme d'onde, des creux de forme d'onde et des passages par zéro ;
      la détermination des au moins les trois premiers cycles sur la base de longueurs de cycle, CL, et d'amplitudes, A, approximatives d'au moins trois cycles supposés présents dans les données sur la base des marqueurs de cycles fractionnaires ; et
      le calcul approximatif d'une périodicité, P, attendue de chacun des trois cycles sur la base des longueurs de cycle, CL, pour chacun des trois cycles ;

   l'exécution d'une sélection basée sur la transformée de périodicité (PT) d'au moins une première périodicité observée à partir de l'analyse de cycle partiel (408), la sélection basée sur la PT comprenant, pour chacune des périodicités, P, attendues des trois cycles :

la projection des données sur un sous-espace de périodicité pour la périodicité, P, attendue respective ;

la vérification pour établir si la projection inclut au moins un pourcentage seuil, T, d'une énergie totale dans un signal constitué par les données, et si tel est le cas, l'acceptation du cycle auquel correspond la périodicité comme un cycle possible présent dans les données ; et

l'estimation d'une longueur de cycle la plus probable sur la base des longueurs de cycle et des amplitudes des cycles possibles identifiés ; et

la détermination de l'activité la plus probable de la au moins une première périodicité à l'aide de l'heuristique (410).

**14.** Procédé selon la revendication 13, dans lequel

l'activité la plus probable est la respiration et la première périodicité est la fréquence respiratoire ; et/ou

dans lequel l'activité la plus probable comprend au moins un élément parmi : un mouvement soudain, un mouvement soutenu, un mouvement lent de la paroi thoracique, un mouvement profond de la paroi thoracique et une forte vibration.

**15.** Procédé selon la revendication 13, dans lequel :

les rapports des amplitudes de la longueur de cycle la plus probable indiquent un indice de qualité de signal ; et/ou

les amplitudes des longueurs de cycle les plus probables et la présence de chaque amplitude par rapport à une région d'intérêt déterminent un indice de qualité de signal pour chaque estimation.

100

**WEARABLE DEVICE 120**
- ACCELEROMETER 122
- GYROSCOPE 123
- PPG SENSOR 124
- ECG SENSOR 125
- TEMPERATURE SENSOR 126
- HUMIDITY SENSOR 127
- LOCATION DETECTION 128
- MICROPROCESSOR 129
- OTHER SENSOR(S) 130

**NETWORK 110**

**MONITORING SYSTEM 140**
- INPUT/OUTPUT INTERFACE 142
- PROCESSOR 144
- DATA STORAGE 146

**MOBILE DEVICE 150**
- INPUT/OUTPUT INTERFACE 152
- PROCESSOR 154
- CARDIAC APPLICATION 156
- DATA STORAGE 158

FIGURE 1

EP 3 958 737 B1

**200**

FIGURE 2

300

BASEBAND PROCESSOR
304

ABB
305

DBB
306

MEMORY
310

OS
312

ASW
314

VSA
315

LPA
316

FA
317

RADIO
302

APPLICATION PROCESSOR
308

USER INTERFACE
318

MEMORY
320

PROTOCOL STACK
321

POWER
MANAGEMENT
322

BATTERY
324

IMAGE CAPTURE
326

COMMUNICATIONS
INTERFACE
328

GNSS RECEIVER
330

332

FIGURE 3

400

START 402

RECEIVE SENSOR DATA 404

PERFORM PARTIAL CYCLE ANALYSIS TO DETERMINE AT LEAST TOP THREE CYCLES USING THE SENSOR DATA 406

PERFORM A PERIODICITY TRANSFORM-BASED SELECTION OF AT LEAST ONE TOP PERIODICITY OBSERVED FROM THE PARTIAL CYCLE ANALYSIS 408

DETERMINE A MOST LIKELY ACTIVITY OF THE AT LEAST ONE TOP PERIODICITY OBSERVED FROM THE PARTIAL CYCLE ANALYSIS USING HEURISTICS 410

MEASURE A SIGNAL QUALITY INDEX FOR AN INCOMING SIGNAL USING THE PRESENCE OR ABSENSE OF PERIODICITY INFORMATION ASSOCIATED WITH THE PARTIAL CYCLE ANALYSIS 412

USE THE SIGNAL QUALITY INDEX TO DETERMINE USER CHARACTERISTICS 414

END 416

FIGURE 4

FIGURE 5

FIGURE 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Towards a Practical Pedestrian Distraction Detection Framework using Wearables. **VINAYAGA-SUR-ESHKANTH, NISHA et al.** 2018 IEEE International Conference on Pervasive Computing and Communications Workshops (PerCom Workshops). IEEE, 19 March 2018, 239-245

- **SETHARES, W.A.** ; **STALEY. T.W.** Periodicity transforms. *IEEE Transactions on Signal Processing*, 01 November 1999, 2953-2964